# EUROPEAN PATENT SPECIFICATION

(11) **EP 2 164 389 B1**
(45) Date of publication and mention of the grant of the patent: **19.06.2013**
(21) Application number: 08763435.8
(22) Date of filing: 01.07.2008
(51) Int. Cl.: A61B 5/0408

(54) **SHIELDED BIOMEDICAL ELECTRODE PATCH**
ABGESCHIRMTE BIOMEDIZINISCHE ELEKTRODENFLÄCHE
PASTILLE D'ELECTRODE BIOMEDICALE BLINDEE

(30) Priority: 06.07.2007 US 948355 P
(43) Date of publication of application: 24.03.2010
(73) Proprietor: Koninklijke Philips Electronics N.V., 5621 BA Eindhoven (NL)
(72) Inventor: GEHMAN, Stacy, Bothell, Washington 98041-3003 (US); HERLEIKSON, Earl, Bothell, Washington 98041-3003 (US); HUGH, Steven, Bothell, Washington 98041-3003 (US); LYSTER, Thomas, Bothell, Washington 98041-3003 (US); SOLOSKO, Thomas, Bothell, Washington 98041-3003 (US)
(74) Representative: van Velzen, Maaike Mathilde
(86) International application number: PCT/IB2008/052645
(87) International publication number: WO 2009/007877

(56) References cited:
- EP-A- 1 752 093
- WO-A-99/56619
- WO-A-03/047427
- WO-A-2007/060609
- WO-A-2007/063436
- GB-A- 2 185 403

## Description

This invention relates to electrode patches for placement on a patient's body to measure physiological signals.

In many biomedical applications, electronics and therapy devices need to be attached to the skin in order to observe and administer therapy or to monitor patient conditions such as blood flow, heart rhythm, and blood oxygen levels. Some common devices include electrodes for generating an electrocardiograph, external defibrillators, pacing devices, transcutaneous nerve stimulation devices, and transdermal drug delivery systems. Often these devices will remain attached to the skin for an extended period of time.

Conductive components in existing electrodes are exposed to electric fields from a number of sources. The modem home and hospital room have many electronic devices and associated cables that may interfere with operation of an electrode. This is particularly true for ambulatory patients. Electrodes affixed to ambulatory patients are particularly susceptible to electric fields caused by clothing-generated static. Because of its inherent capacitance the electrode will generate current in response to these electric fields that will interfere with the accuracy of signals sent from and received by the electrode.

WO 99/56619 A discloses an electrocardiographic electrodes holder which has a body with a first plane, and a second plane for respectively engaging six electrocardiographic electrodes in accepting holes, six conductive recording lines, and six grounding lines for shielding the respective recording lines extending from the holes to the terminal for each of the lines.

The problem of external electric fields is particularly pronounced where a patient is ambulatory. Electrodes worn by a patient beneath clothing are susceptible to noise created by clothing-generated static, which creates very large amplitude noise that may interfere with or completely obscure the biomedical signal being monitored.

In view of the foregoing it would be advantageous to provide an electrode that is suitable for attaching to a patient's skin and is shielded from ambient electric fields.

Relevant prior art is disclosed by WO 2007/060 609 A2. The invention is defined by claim 1.

One aspect of the invention includes a biomedical electrode patch including a film layer having a conductive shield and a contact portion printed on the upper surface. The shield and contact portion are isolated from one another on the upper surface. The contact portion extends through the film layer and contacts an electrode formed of a material suitable for conducting electrical signals from a patient's skin.

In an example, however not forming part of the invention, the film layer includes an electrode portion and an elongated tab portion extending from the electrode portion. Contact portions are printed on the lower surface of the electrode portion. Traces printed on the lower surface of the trace portion extend onto the electrode portion and connect with the contact pads. A shield layer is printed on the upper surface of the electrode and trace portions of the film layer.

In an example, however not forming part of the invention, a ground trace is formed on the lower surface of the trace portion and is coupled through the film layer to the shield layer. A trace shield layer is printed on the lower surface of the trace portion over the traces. A non-conductive layer separates the trace shield layer from the traces, except for the ground trace that is electrically coupled to the trace shield layer.

In another aspect of the invention, a receptacle secures to the shield layer and includes a shield contact and a contact portion contact. A plug having contacts corresponding to the shield contact and contact portion contact is positionable within the receiver.

In the drawings:

FIGURE 1 is an exploded view of a biomedical patch in accordance with an embodiment of the present invention.

FIGURE 2 is a top plan view of a conductive layer bearing a shield layer in accordance with an embodiment of the present invention.

FIGURE 3 is a top plan view of a via formed in the conductive layer in accordance with an embodiment of the present invention.

FIGURE 4 is a bottom plan view of the conductive layer bearing contact portions in accordance with an embodiment of the present invention.

FIGURE 5 is a top plan view of the conductive layer bearing a dielectric layer in accordance with an embodiment of the present invention.

FIGURE 6 is a top plan view of the conductive layer bearing an adhesive layer.

FIGURE 7 is a top plane view of the conductive layer bearing a non-isotropically conductive layer in accordance with an embodiment of the present invention.

FIGURE 8 is a top plan view of a conductive layer having a holster secured thereto.

FIGURE 9 is a top plan view of an alternative example of a shielded circuit layer for an electrode patch.

FIGURE 10 is a bottom plan view of the shielded circuit layer for the electrode patch of FIGURE 9.

FIGURE 11 is an exploded view of an assembly including the electrode patch of FIGURE 9.

FIGURE 12 is an exploded view of an electrode patch in accordance with an embodiment of the present invention.

FIGURE 13 is an exploded view of the electrode patch of FIGURE 12 viewed from the opposite direction.

FIGURE 14 is a top plan view of the electrode patch of FIGURE 12.

FIGURE 15 is an exploded view of another alternative example of an electrode patch.

FIGURE 16 is a top plan view of the electrode patch of FIGURE 15.

FIGURE 17 is a bottom plan view of the electrode patch of FIGURE 15.

FIGURE 18 is an exploded view of another alternative example of a shielded electrode patch.

FIGURE 19 is an exploded view of another alternative example of an electrode patch.

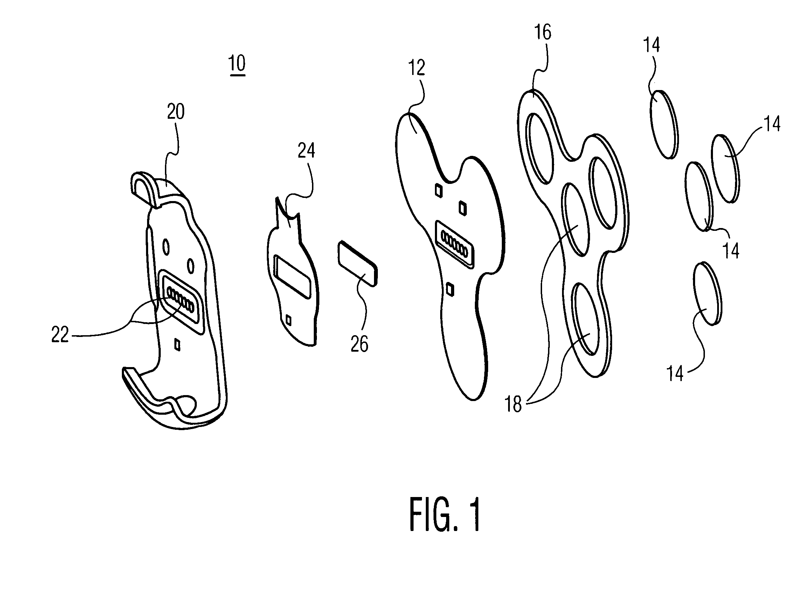

Referring to FIGURE 1, a biomedical electrode patch 10 includes a conductive layer 12 electrically coupled to one or more electrodes 14 formed of a material suitable for conducting electrical signals to and from a patient's skin. In a preferred embodiment the electrodes 14 are formed of hydrogel, such as Axelgaard's Ag602. The electrode 14 may be formed in sheets and cut to shape or cured in place by means of UV curing or a like process. The patch 10 may include a foam layer 16 having apertures 18 sized to receive the electrodes 14. A surface of the foam layer 16 adjacent a conductive layer 12 may bear an adhesive such as MacTac MP597 serving to secure the foam layer 16 to the conductive layer 12. The foam layer 16 is typically somewhat compressible and can have a thickness slightly less than that of the electrodes 14 such that the electrodes project slightly from the apertures 18. A holster 20 having a plurality of electrical contacts 22 mounts to the conductive layer opposite the electrodes 14. The contacts 22 provide an electrical connection that conducts signals between the patch and a monitoring device or other device (not shown).

The holster 20 is secured to the conductive layer 12 by means of a layer 24 of adhesive, such as a pressure sensitive adhesive (PSA). A layer 26 of conductive PSA may be used to conduct signals from the contacts 22 to the conductive layer 12.

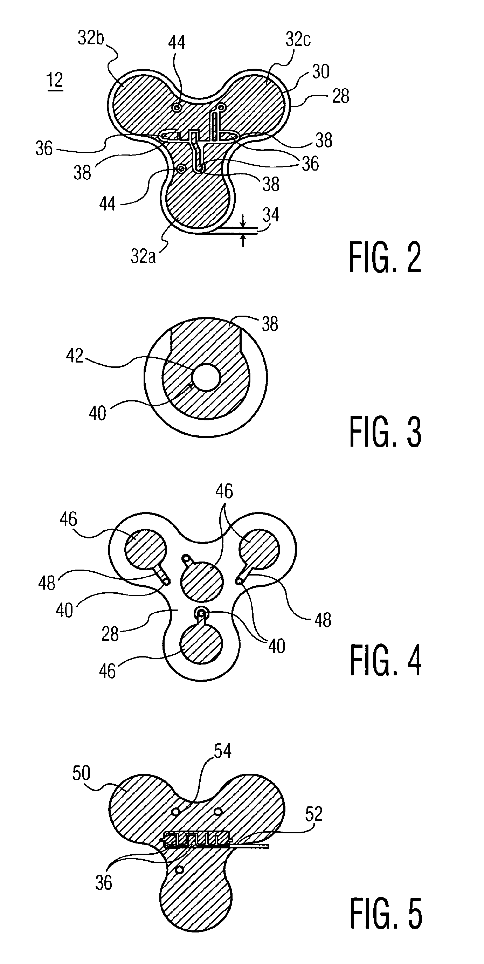

FIGURE 2 illustrates an upper surface of the conductive layer 12. The conductive layer 12 in some embodiments may include a film layer 28 made of a non-conductive material, for example, polyester, or a like material. A shield 30 is formed on the conductive layer 12 and may include portions 32a-32c extending over positions corresponding to the electrodes 14. In some embodiments, a dielectric layer is interposed between the shield 30 and the film layer 28. In some examples, the shield 30 extends to within a distance 34 from the edge of the film layer 28. In an example, the distance 34 is about 0.635 mm (0.025 inches). Providing a non-conductive area around the perimeter of the film layer 28 may reduce the likelihood of undesirable electrical contact between the shield 30 and a patient's skin which is best avoided at times such as when the patient's skin is wet.

The shield 30 may be formed of conductive ink printed on the upper surface of the film layer 28. Suitable inks include silver inks and silver/silver chloride inks, though other conductive inks may be used. The ink may be applied to the film layer 28 by any method known in the art.

A number of contact pads 36 are also formed on a first surface of the film layer 28. The contact pads 36 of the illustrated embodiment are aligned in a row, however other arrangements are possible. The contact pads 36 are preferably arranged in correspondence with conductive members of a device coupling to the patch 10. In the illustrated embodiment, the contact pads 36 are positioned corresponding to the contacts 22 mounted to the holster 20.

A non-conductive gap is formed between the shield 30 and the contact pads 36. However, in some examples, a number of the contact pads 36 are separated from the shield 30 and each other by a nonconductive gap whereas one or more of the contact pads 36 is electrically coupled to the shield 30 in order to provide a connection between the shield 30 and, for example, the ground of a monitoring device in order to reduce common mode defects in signals output from the patch 10. In some examples, the shield 30 does not extend over the contact pads 36. In such examples, the connector or device positioned over the contact pads may provide electrical shielding for the contact pads 36.

Referring to FIGURE 3 while still referring to FIGURE 2, traces 38 may connect a number of the contact pads 36 to vias 40 providing a conductive path from a first side of the film layer 28 to a second side opposite the first side. The contact pads 36, traces 38, and vias 40 may be formed of conductive ink, such as silver ink, silver/silver chloride ink, or the like applied by any means known in the art. In the illustrated example, the vias 40 are embodied as holes 42 extending through the film layer and having conductive ink printed on both sides of the film layer 28 around and through the through-holes 42 such that the ink on one side of the film layer is in contact with ink on the other to form an electrical connection.

In some examples, alignment marks 44 are printed on the film 28. The alignment marks 44 may be formed as discrete areas of conductive ink separated from the shield 30 by unprinted areas encircling the alignment marks 44.

Referring to FIGURE 4, a number of pads 46 are formed on the lower surface of the film layer 28. The pads 46 are typically positioned in correspondence with the electrodes 14 and may have approximately the same area as the electrodes 14. Traces 48 are also formed on the lower surface of the film layer 28, with each trace 48 extending from a via 40 to one of the pads 46. In an alternative embodiment, the vias 40 are co-located with the pads 46 such that traces 48 are unnecessary.

Referring to FIGURE 5, in some embodiments, a dielectric layer 50 is formed over the shield 30 in order to reduce artifacts caused by finger touches and other electrical contact with the shield 30. The dielectric layer 50 may define an opening 52 over the pads 36 such that the pads 36 are exposed. The dielectric layer 50 may also define openings 54 over the alignment marks 44 such that they remain visible.

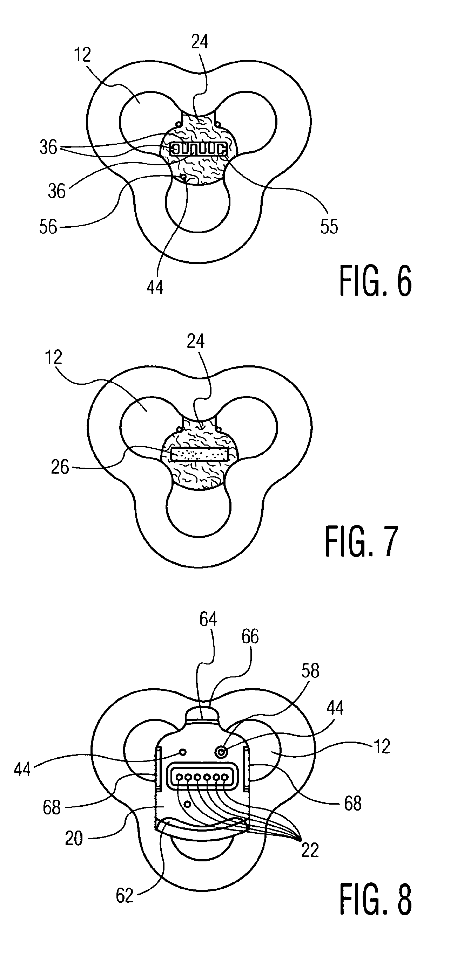

Referring to FIGURE 6, the adhesive layer 24 is secured over the shield layer 30, or the dielectric layer 50 of the conductive layer 12. The adhesive layer 24 may define one or more apertures 55 exposing the contact pads 36. Various types of adhesive may be used to form the adhesive layer 24. In one example 3M's 1524 medical grade PSA is used. The adhesive layer 24 may further define openings 56 exposing the alignment marks 44. Referring to FIGURE 7, a non-isotropically conductive adhesive layer 26 is positioned within the opening 55. The non-isotropic adhesive layer 26 may be formed of a pressure-sensitive adhesive that conducts electricity through the thickness of the layer 26 but does not substantially conduct electricity parallel to the layer 26. In some examples, the adhesive layer 24 is eliminated and the layer 26 is enlarged beyond the area of the contact pads to provide sufficient adhesive contact.

Referring to FIGURE 8, a holster 20 is adhered to the adhesive layer 24 and conductive layer 26. The holster 20 may include openings 58 arranged in a pattern corresponding to one or more of the alignment marks 44. When securing the holster 20 to the adhesive layer a manufacturer may align the openings 58 with the alignment marks 44 to position the holster 20 such that the contacts 22 are aligned with the pads 36. The holster 20 may include a panel (not shown) mounting the contacts 22. In one example, the panel 60 is formed of silicone, or other resilient polymer. One or more locking tabs 62, 64 may be formed on the holster 20 to retain a monitoring device or other connector within the holster. A release tab 66 may be secured to the tab 64 to enable a user to bend the tab 64 out of the way to remove a plug or other connector. Ridges 68 extending along the sides of the holster 20 may serve to align a plug, or other connector, within the holster 20 and hinder lateral movement in order to maintain the monitoring device or other connector aligned with the contacts 22.

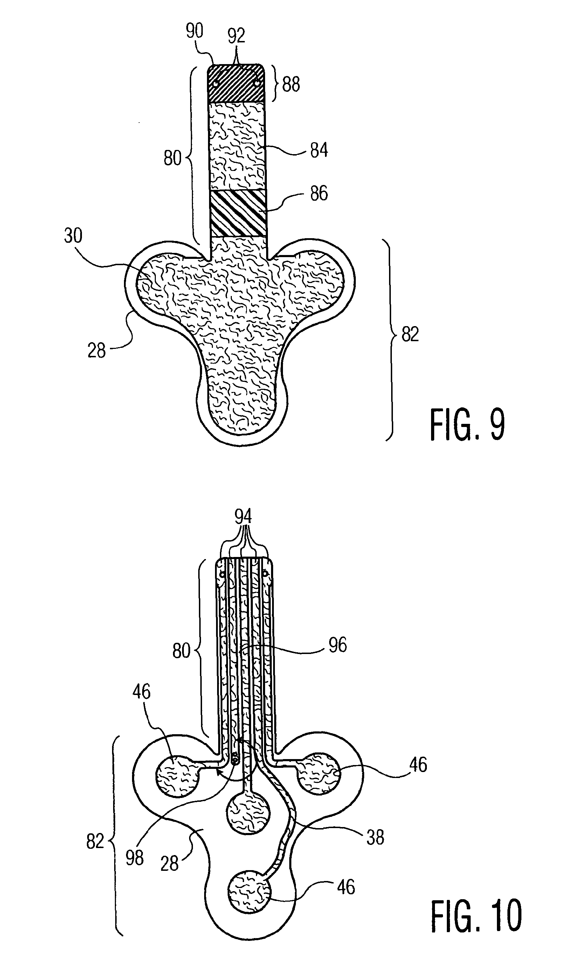

Referring to FIGURE 9, in an alternative example of an electrode patch 10, a film layer 28 includes a tab portion 80 and an electrode portion 82. In some examples, the shield 30 includes a shield tab portion 84 formed over the tab 80. The shield tab portion 84 may be electrically coupled to the shield 30. A dielectric layer may be formed between the film layer 28 and shield 30, including the shield tab portion 84. A transfer adhesive layer 86 may secure to the shield tab portion 82 or electrode portion 80 such that the tab portion 80 may be folded over and secured to the electrode portion 82. The transfer adhesive layer 86 is typically a double sided adhesive film and may include a pressure sensitive adhesive.

A terminal portion 88 of the tab portion 80 may include a stiffener 90 to facilitate coupling of the terminal portion 88 to the holster 20. The stiffener 90 may include one or more alignment holes 92 to facilitate positioning of the terminal portion 88 within the holster 20. The alignment holes may also extend through the film layer 28.

Referring to FIGURE 10, the lower surface of the tab portion 80 may include trace tab portions 94 extending from the traces 38 coupled to the pads 46. A ground trace 96 may also be formed on the lower surface of the tab portion 80. One or more vias 98 may extend between the ground trace 96 and the shield tab portion 84. In the illustrated example, the vias 98 are embodied as holes formed in the film layer having conductive ink printed thereon such that conductive ink printed over the upper surface of the film layer contacts ink printed over the lower surface of the film layer 28. Other techniques for conductively connecting the ground trace 96 to the shield tab portion 84 or the shield 30 can be used as well.

A dielectric layer may be printed over the trace tab portions 94 and the ground trace 96. In other examples, a lower shield layer may be formed over the dielectric layer. The lower shield layer can be coupled to the shield layer 30 by the ground trace 96 and vias 98. An additional dielectric layer may be formed over the lower shield layer.

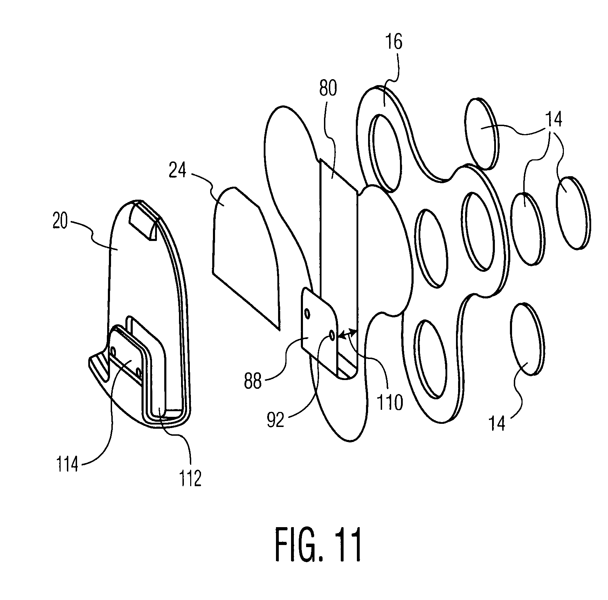

Referring to FIGURE 11, the tab portion 80 may be folded as illustrated such that the terminal portion 88 is doubled back over the tab portion 80 and is positioned a distance 110 above the tab portion 80. The adhesive layer 24 adhering the holster 20 to the film layer 28 may extend over the tab portion 80 and secure to the shield 30, or a dielectric layer formed over the shield 30 on either side of the tab portion 80. The holster 20 may include an aperture 112 through which the tab portion 80 extends. A projection 114 extends over the aperture 112 and is secured to the terminal portion. Pins may extend downwardly from the projection 114 and into the alignment holes 92 in the terminal portion 88 and stiffener 90.

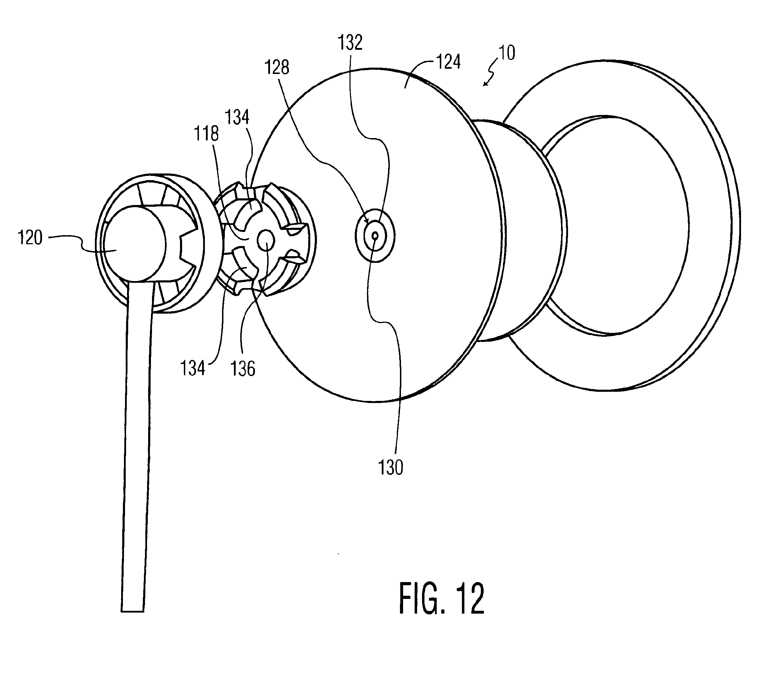

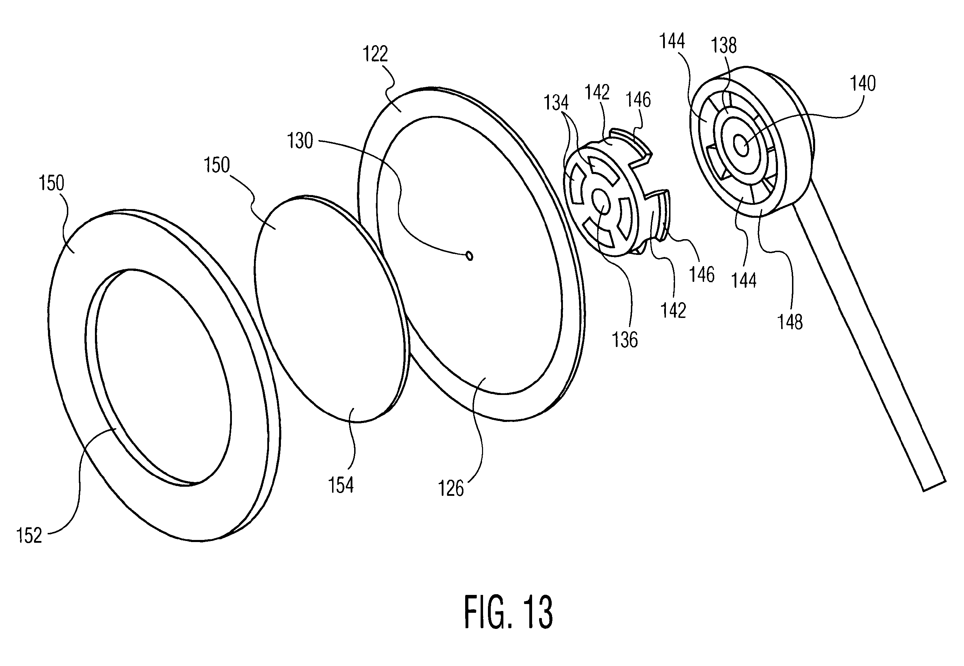

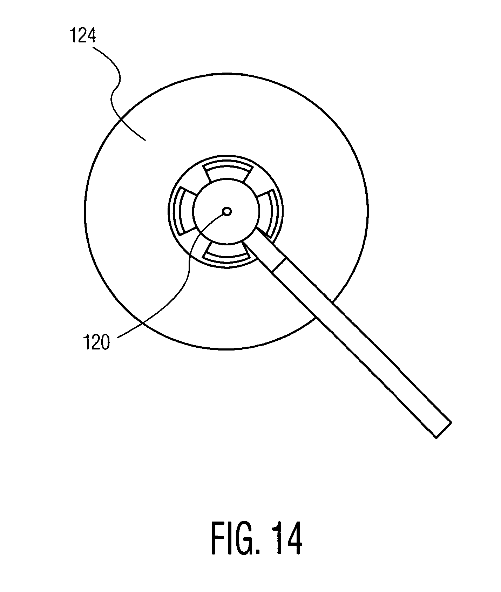

Referring to FIGURES 12, 13, and 14, in an alternative embodiment, a patch 10 includes a receptacle 118 that receives a plug 120, or other connector in order to electrically couple the patch 10 to a monitoring apparatus, or other device. The receptacle 118 is secured to a film layer 122 having a shield 124 printed on an upper surface thereof and a contact pad 126 printed on a lower surface. A via 128 extends through the film layer and is electrically coupled to the contact pad 126. The via 128 may be conductive ink printed on either side of an aperture 130 extending through the film layer such that a conductive path is created. An unprinted region 132 may be formed around the via 128 such that the shield 124 is not electrically coupled to the via 128.

The receptacle 118 includes one or more receptacle shield contacts 134 and at least one receptacle via contact 136. In the illustrated example, the shield contacts are located concentrically around the pad contact 136. The contacts 134, 136 may be formed of a conductive adhesive polymer and may extend through the receptacle 118. In use, the receptacle is secured to the shield 124 and via 128 by means of the adhesive contacts 134, 136. The connector 120 includes a connector shield contact 138 and a connector via contact 140 in positions corresponding to the contacts 134, 136 such that the contacts 138 electrically coupled to the contacts 134 and the contacts 140 electrically coupled to the contacts 136 when the connector 120 is positioned within the receptacle 118. The receptacle 118 may include one or more locking tabs 142 engaging apertures 144 formed on the connector 120. The locking tabs 142 may include hooks 146 engaging a rim 148 surrounding the apertures 144.

As with other examples of the patch 10, a foam layer 150 may secure to the lower surface of the film layer 122. The surface of the foam layer adjacent the film layer 122 may bear an adhesive such as MacTac MP597. The foam layer 150 may include an aperture 152 that receives an electrode 154 formed of a material such as hydrogel. A retaining layer and backing layer may also secure to patch 10 as in other embodiments described herein.

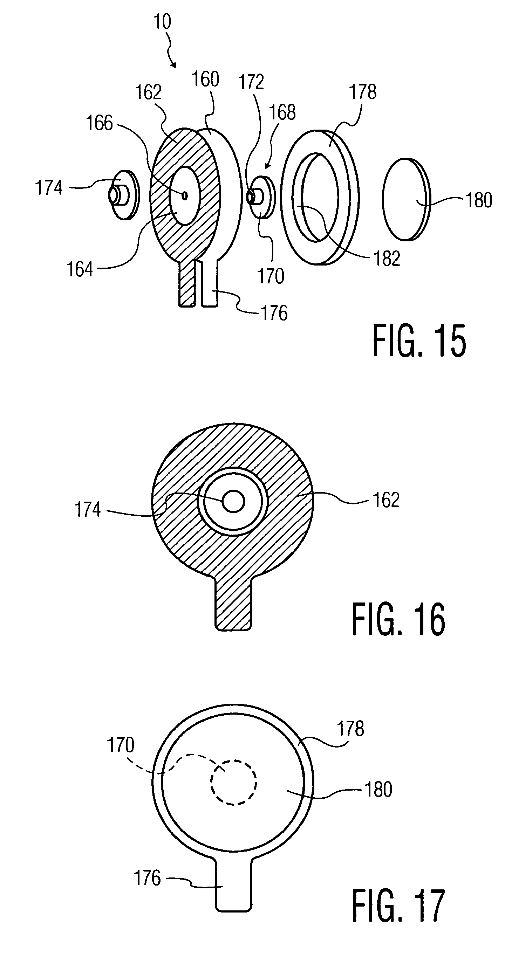

Referring to FIGURES 15 through 17, the biomedical electrode patch 10 includes a film layer 160 having a shield portion 162 printed or laminated on an upper surface thereof. In the illustrated example, the shield portion 162 has an annular shaped portion surrounding a dielectric portion 164 printed or laminated on the film layer. An aperture 166 extends through the dielectric portion 164 and the film layer 160. A fastener 168 secures within the aperture 166. In the illustrated example, the fastener 168 includes an eyelet 170 having a post 172 and a cap 174 sized to receive the post 172. The fastener 168 may be formed of a conductive plastic or may be bear a conductive coating or a conductive ink such as silver/silver chloride ink. The film layer 160 may include a tab portion 176 extending outwardly therefrom. The shield portion 162 may extend over the tab portion 176. In some examples, a connector secures to the tab portion 176 in order to electrically couple the shield portion 162 to a monitoring device.

A foam layer 178 may secure to the lower surface of the film layer 160 and a conductive interface 180 may be positioned within an aperture 182 in the foam layer in electrical contact with the fastener 168. In the illustrated example, the conductive interface 180 is formed of hydrogel and may have a thickness slightly greater than that of the foam layer 178 such that it projects slightly.

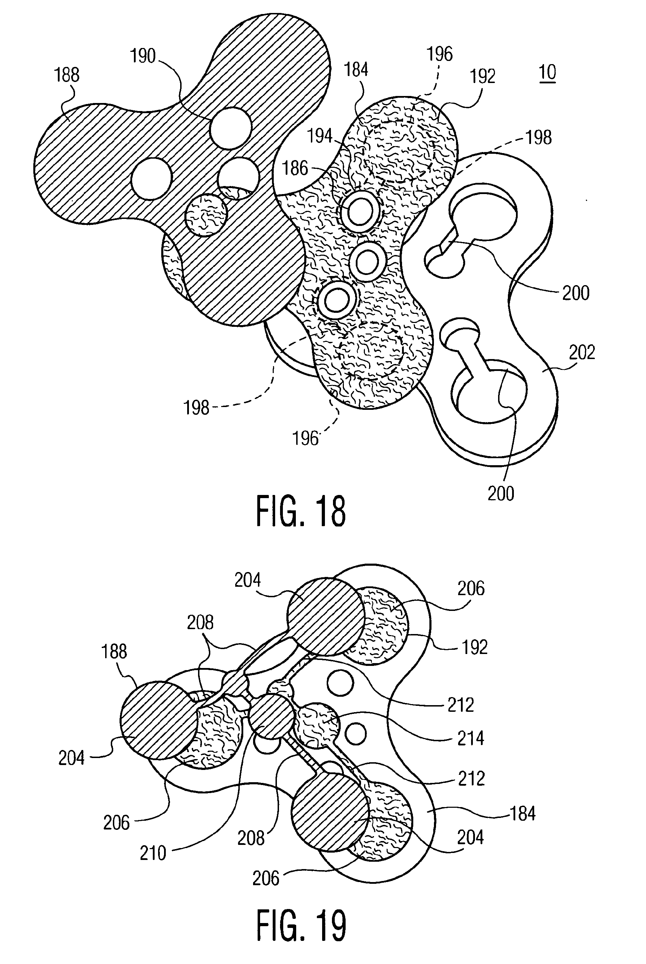

Referring to FIGURE 18, a biomedical electrode patch 10 includes a film layer 184 having a number of apertures 186 formed therein. A shield layer 188 formed of a conductive material is printed or laminated onto the film layer 184. The shield layer 188 includes apertures 190 corresponding in position to the apertures 186. The apertures 190 in the shield layer 188 may be slightly larger than the apertures 186. In the illustrated example, a dielectric layer 192 is positioned between the shield layer 188 and the film layer 184. The dielectric layer 192 may include apertures 194 corresponding in position to the apertures 186 and being slightly larger than the apertures 186. The dielectric layer 192 may advantageously reduce the capacitance between the shield layer 188 and electrodes 196 positioned below the film layer 184 for contacting a patient's skin.

Traces 198 may extend from the electrodes 196 across the apertures 186 such that a connector may secure to the patch 10 and electrically contact the electrodes 194. The traces 198 and electrodes 196 may be formed of hydrogel. Each trace 198 may be monolithically formed with one of the electrodes 196. The electrodes 196 and traces 198 may fit within apertures 200 within a foam layer 202 secured to the lower surface of the film layer 184.

Referring to FIGURE 19, in an alternative example of the biomedical electrode patch of FIGURE 18, the shield layer 188 includes portions 204 corresponding in size and position to the electrodes 196 and covering the electrodes and traces on the opposite side of the printed film circuit layer. The dielectric layer 192 may likewise include dielectric portions 206 corresponding in size and position to the electrodes 196. The electrode-covering portions 204 of the shield layer 186 may be connected to one another by traces 208. In some examples, a contact pad 210 is connected to the traces 208. The contact pad 210 may electrically contact a connector which also electrically contacts the electrodes 196. The dielectric layer 192 may overlap the printed conductive trace portions 212 on the film layer 184. The embodiment of FIGURE 19 may advantageously be more flexible than the example of FIGURE 18 inasmuch as portions of the shield layer 188 and dielectric layer 196 have been removed.

## Claims

1. A biomedical electrode (10,) comprising:
a film layer (28) having an upper surface and a lower surface, the upper surface bearing a conductive layer having a shield portion (30, 124) and at least one contact portion (40, 128) comprising a conductive material,
an electrode layer (126) configured to conduct electrical signals from a patient's skin, the at least one contact portion extends through the film thereby providing a conductive path from the upper surface to the lower surface;
the electrode layer is printed on the lower surface and contacts the contact portion through the film, and wherein the contact portion and shield portion define a non-conductive gap therebetween **characterized in that**
the shield portion substantially surrounds the contact portion and covers the electrode layer printed on the lower surface of the film layer..

2. The electrode of claim 1, wherein the electrode layer comprises a plurality of electrodes (46), wherein each of the at least one contact portions is positioned over one of the plurality of electrodes or traces (48) extending from the electrodes on the electrode layer, and wherein the conductive layer further comprises a plurality of connection pads (36) located adjacent one another and a plurality of traces (38) each extending from one of the connection pads to one of the at least one contact portions.

3. The electrode of claim 2, wherein the shield portion is electrically coupled to one of the plurality of connection pads.

4. The electrode of claim 3, further comprising a non-isotropically conductive layer (26) positioned over the plurality of connection pads.

5. The electrode of claim 4, further comprising a receptacle secured to the upper surface, the receptacle having a plurality of conductive members positioned in contact with the non-isotropic conductive layer each being positioned over one of the plurality of contact pads.

6. The electrode of claim 3, further comprising a receptacle (118) secured to the upper surface, the receptacle having a plurality of conductive members positioned in direct contact with the plurality of connection pads.

7. The electrode of claim 1, further comprising a compressible layer attached to the lower surface (16), the compressible layer having apertures formed therein to receive hydrogel which contacts the electrode layer.

8. The electrode of claim 7, wherein the compressible layer has an undeformed thickness less than the thickness of the hydrogel or electrically conductive skin-contacting layer.

9. The electrode of claim 1, wherein the electrode layer further comprises a skin-contacting hydrogel.

10. The electrode of claim 1, further comprising a retention seal secured to the electrode layer and having at least one aperture positioned over the electrode layer.

11. The electrode of claim 1, further comprising a first dielectric layer formed over the shield layer.

12. The electrode of claim 11, further comprising a second dielectric layer formed between the shield layer and the film layer.

13. The electrode according to claim 1 for use in a biomedical electrode patch.

14. The electrode of claim 1, further comprising
a receptacle secured to the upper surface, the receptacle having an inner receiving surface and a conductive member extending from the inner receiving surface to the conductive layer.

## Patentansprüche

1. Biomedizinische Elektrode (10), die Folgendes umfasst:
eine Filmschicht (28) mit einer oberen Oberfläche und einer unteren Oberfläche, wobei die obere Oberfläche eine leitende Schicht mit einem Abschirmungsteil (30, 124) und mindestens einem ein leitendes Material umfassenden Kontaktteil (40, 128) trägt,
eine Elektrodenschicht (126), die konfiguriert ist, um elektrische Signale von der Haut eines Patienten zu leiten, wobei sich der mindestens eine Kontaktteil durch den Film erstreckt und dadurch einen leitenden Pfad von der oberen Oberfläche zu der unteren Oberfläche schafft;
wobei die Elektrodenschicht auf die untere Oberfläche aufgedruckt ist und durch den Film mit dem Kontaktteil in Kontakt ist, und wobei der Kontaktteil und der Abschirmungsteil eine nicht-leitende Lücke dazwischen definieren, **dadurch gekennzeichnet, dass**
der Abschirmungsteil im Wesentlichen den Kontaktteil umgibt und die auf die untere Oberfläche der Filmschicht aufgedruckte Elektrodenschicht abdeckt.

2. Elektrode nach Anspruch 1, wobei die Elektrodenschicht eine Vielzahl von Elektroden (46) umfasst, wobei jeder der mindestens einen Kontaktteile über einer von der Vielzahl von Elektroden oder Bahnen (48) positioniert ist, die ausgehend von den Elektroden auf der Elektrodenschicht verlaufen, und wobei die leitende Schicht weiterhin eine Vielzahl von nebeneinander angeordneten Verbindungsflächen (36) umfasst und sich eine Vielzahl von Bahnen (38) von einer der Verbindungsflächen zu einem der mindestens einen Kontaktteile erstreckt.

3. Elektrode nach Anspruch 2, wobei der Abschirmungsteil elektrisch mit einer von der Vielzahl von Verbindungsflächen gekoppelt ist.

4. Elektrode nach Anspruch 3, weiterhin mit einer nicht-isotropischen leitenden Schicht (26), die über der Vielzahl von Verbindungsflächen angeordnet ist.

5. Elektrode nach Anspruch 4, weiterhin mit einer Aufnahme, die an der oberen Oberfläche befestigt ist, wobei die Aufnahme eine Vielzahl von leitenden Elementen hat, die in Kontakt mit der nicht-isotropischen leitenden Schicht positioniert sind, wobei jedes über einer von der Vielzahl von Kontaktflächen positioniert ist.

6. Elektrode nach Anspruch 3, weiterhin mit einer an der oberen Oberfläche befestigten Aufnahme (118), wobei die Aufnahme eine Vielzahl von leitenden Elementen hat, die in direktem Kontakt mit der Vielzahl von Verbindungsflächen positioniert sind.

7. Elektrode nach Anspruch 1, weiterhin mit einer an der unteren Oberfläche (16) angebrachten komprimierbaren Schicht, wobei die komprimierbare Schicht Aperturen hat, die darin ausgebildet sind, um Hydrogel aufzunehmen, das die Elektrodenschicht kontaktiert.

8. Elektrode nach Anspruch 7, wobei die komprimierbare Schicht eine unverformte Dicke hat, die geringer ist als die Dicke des Hydrogels oder der elektrisch leitenden, mit der Haut in Kontakt befindlichen Schicht.

9. Elektrode nach Anspruch 1, wobei die Elektrodenschicht weiterhin ein mit der Haut in Kontakt befindliches Hydrogel umfasst.

10. Elektrode nach Anspruch 1, weiterhin mit einer Rückhaltedichtung, die an der Elektrodenschicht befestigt ist und mindestens eine Apertur hat, welche über der Elektrodenschicht positioniert ist.

11. Elektrode nach Anspruch 1, weiterhin mit einer ersten dielektrischen Schicht, die über der Abschirmungsschicht ausgebildet ist.

12. Elektrode nach Anspruch 11, weiterhin mit einer zweiten dielektrischen Schicht, die zwischen der Abschirmungsschicht und der Filmschicht ausgebildet ist.

13. Elektrode nach Anspruch 1 zur Verwendung in einem biomedizinischen Elektrodenpflaster.

14. Elektrode nach Anspruch 1, weiterhin mit
einer an der oberen Oberfläche befestigten Aufnahme, wobei die Aufnahme eine innere Aufnahmeoberfläche hat und sich ein leitendes Element von der inneren Aufnahmeoberfläche aus zur leitenden Schicht erstreckt.

## Revendications

1. Électrode biomédicale (10) comprenant :
une couche de film (28) ayant une surface supérieure et une surface inférieure, la surface supérieure portant une couche conductrice ayant une partie de blindage (30, 124), et au moins une partie de contact (40, 128) comprenant un matériau conducteur,
une couche d'électrode (126) configurée de façon à conduire des signaux électriques provenant de la peau d'un patient, ladite au moins une partie de contact s'étendant à travers le film, en fournissant ainsi un chemin conducteur depuis la surface supérieure à la surface inférieure ;
la couche d'électrode étant imprimée sur la surface inférieure et entrant en contact avec la partie de contact à travers le film, dans laquelle la partie de contact et la partie de blindage définissent un intervalle non-conducteur entre elles, **caractérisée en ce que**
la partie de blindage entoure sensiblement la partie de contact et couvre la couche d'électrode imprimée sur la surface inférieure de la couche de film.

2. Électrode selon la revendication 1, dans laquelle la couche d'électrode comprend une pluralité d'électrodes (46), dans laquelle chacune desdites au moins une partie de contact est positionnée sur une parmi la pluralité d'électrodes ou traces (48) s'étendant depuis les électrodes sur la couche d'électrode, et dans laquelle la couche conductrice comprend en outre une pluralité de plaquettes de connexion (36) situées de manière adjacente les unes aux autres et une pluralité de traces (38) s'étendant chacune d'une des plaquettes de connexion à l'une desdites au moins une partie de contact.

3. Électrode selon la revendication 2, dans laquelle la partie de blindage est électriquement raccordée à l'une parmi la pluralité des plaquettes de connexion.

4. Électrode selon la revendication 3, comprenant en outre une couche conductrice de manière non isotrope (26), positionnée sur la pluralité de plaquettes de connexion.

5. Électrode selon la revendication 4, comprenant en outre un réceptacle fixé à la surface supérieure, le réceptacle ayant une pluralité d'éléments conducteurs positionnés en contact avec la couche conductrice non-isotrope, chacun étant positionné sur une parmi la pluralité des plaquettes de contact.

6. Électrode selon la revendication 3, comprenant en outre un réceptacle (118), fixé à la surface supérieure, le réceptacle ayant une pluralité d'éléments conducteurs positionnés en contact direct avec la pluralité de plaquettes de connexion.

7. Électrode selon la revendication 1, comprenant en outre une couche compressible fixée à la surface inférieure (16), la couche compressible ayant des ouvertures ménagées à l'intérieur pour recevoir de l'hydrogel, qui entre en contact avec la couche d'électrode.

8. Électrode selon la revendication 7, dans laquelle la couche compressible a une épaisseur non déformée inférieure à l'épaisseur de l'hydrogel ou de la couche en contact avec la peau électriquement conductrice.

9. Électrode selon la revendication 1, dans laquelle la couche d'électrode comprend en outre un hydrogel en contact avec la peau.

10. Électrode selon la revendication 1, comprenant en outre un joint de rétention fixé à la couche d'électrode et ayant au moins une ouverture positionnée sur la couche d'électrode.

11. Électrode selon la revendication 1, comprenant en outre une première couche diélectrique formée sur la couche de blindage.

12. Électrode selon la revendication 11, comprenant en outre une seconde couche diélectrique formée entre la couche de blindage et la couche de film.

13. Électrode selon la revendication 1 à utiliser dans un patch à électrode biomédical.

14. Électrode selon la revendication 1, comprenant en outre :
un réceptacle fixé à la surface supérieure, le réceptacle ayant une surface de réception interne et un élément conducteur s'étendant depuis la surface de réception interne vers la couche conductrice.
